# EUROPEAN PATENT SPECIFICATION

(11) **EP 2 418 961 B1**
(45) Date of publication and mention of the grant of the patent: **13.12.2017**
(21) Application number: 10764968.3
(22) Date of filing: 12.04.2010
(51) Int. Cl.: A23K 20/163

(54) **MILK OLIGOSACCHARIDE COMPOSITIONS AND USE THEREOF IN TREATING INFECTION IN ANIMALS**
MILCH-OLIGOSACCHARID-ZUSAMMENSETZUNG UND IHRE VERWENDUNG BEI DER BEHANDLUNG VON INFEKTIONEN BEI TIEREN
COMPOSITIONS D'OLIGOSACCHARIDES DE LAIT ET LEUR UTILISATION DANS LE TRAITEMENT D'UNE INFECTION CHEZ DES ANIMAUX

(30) Priority: 13.04.2009 US 168674 P
(43) Date of publication of application: 22.02.2012
(73) Proprietor: Instituto Nacional De Ciencias Medicas Y Nutricion, Mexico, D.F. 14000 (MX); The General Hospital Corporation, Boston, MA 02114 (US); Children's Hospital Medical Center, Cincinnati, OH 45229-3039 (US)
(72) Inventor: NEWBURG, David S., Massachusetts 02460 (US); RUIZ-PALACIOS, Guillermo M., Mexico City, 14460 (MX); MORROW, Ardythe L., Ohio 42513 (US)
(74) Representative: Chajmowicz, Marion
(86) International application number: PCT/US2010/030728
(87) International publication number: WO 2010/120682

(56) References cited:
- WO-A2-2005/055944
- US-A- 5 700 671
- US-A- 6 126 961
- US-A1- 2002 019 991
- DAI D ET AL: "Role of oligoaccharides and glyoconjugates in intestinal host defense", JOURNAL OF PEDIATRIC GASTROENTEROLOGY AND NUTRITION, LIPPINCOTT WILLIAMS WILKINS, INC, US, vol. 30, no. Suppl. 2, 1 January 2000 (2000-01-01), pages S23-S33, XP008082299, ISSN: 0277-2116

## Description

### BACKGROUND OF THE INVENTION

Human infants are vulnerable to pathogens due to their immature immune systems. They rely heavily on human milk for protection against infections. It is known that human milk contains various components conferring immunologic benefits. Young animals, on the other hand, often do not receive the same kinds of immune protection from the milk of their mothers. Indeed, non-mammals do not normally consume milk during their infancy.

Like in humans, infection is a serious health problem in animals (especially livestock), resulting in enormous economic consequences. There is a need to develop a method for controlling infection in animals.

US patent application published as US2002/019991 discloses compositions containing an alpha 1,2- fucose linkage for treating infections caused by *E. coli* and *V. cholerae.*

### SUMMARY OF THE INVENTION

In one aspect, the present invention features a food composition for use in treating an infection caused by Clostridium perfringens in an animal, which includes an animal foodstuff and a milk-derived oligosaccharide or a glycoconjugate containing the oligosaccharide. The oligosaccharide, preferably derived from human milk, contains a first sugar unit (i.e., a fucose, a galactose, a mannose, or a sialic acid) linked to a second sugar unit (i.e., a galactose, a glucose, a mannose, or an N-acetylglucosamine). The first sugar unit is located at a non-reducing end of the oligosaccharide. In one example, the oligosaccharide is a linear molecule having one non-reducing end and one reducing end. In another example, it is a branched molecule having multiple non-reducing ends and one reducing end. When the oligosaccharide has two non-reducing ends, the sugar unit at one non-reducing end can be fucose and that at the other non-reducing end can be fucose, galactose, or sialic acid, or alternatively, the sugar unit at one non-reducing end is sialic acid and that at the other non-reducing end is galactose or sialic acid. The sugar unit at the reducing end can be a glucose or an N-acetylglucosamine.

In one example, the composition of this invention contains two or more different milk-derived oligosaccharides as described above. In one example, all of the oligosaccharides are attached to a backbone molecule (e.g., a lipid, a peptide, or a carbohydrate) to form a glycoconjugate.

In another aspect, this invention features a veterinary pharmaceutical composition (i.e., a pharmaceutical composition formulated for animal use) for use in treating an infection caused by Clostridium perfringens in an animal, containing any of the above mentioned milk-derived oligosaccharides or glycoconjugates and a pharmaceutically acceptable carrier.

In yet another aspect, it is described a method for treating infection in an animal that needs the treatment by administering to the animal an effective amount of one or more of the above-described milk-derived oligosaccharides or glycoconjugates. The infection to be treated by this method is caused by *Clostridium perfringens.* The term "animal" refers to non-human vertebrates (e.g., mammals, birds, fishes, reptiles, and amphibians), including both young and adult ones. Examples include, but are not limited to, cat, cattle, cow, dog, goat, horse, pig, rabbit, rodent, mink, sheep, chicken, duck, goose, turkey, ostrich, emu, swan, peafoul, pheasant, partridge, and guineafowl.

The details of one or more examples of the invention are set forth in the description below. Other features or advantages of the present invention will be apparent from the following detailed description of several embodiments, and also from the appended claims.

### BRIEF DESCRIPTION OF THE DRAWINGS

The drawing is first described:

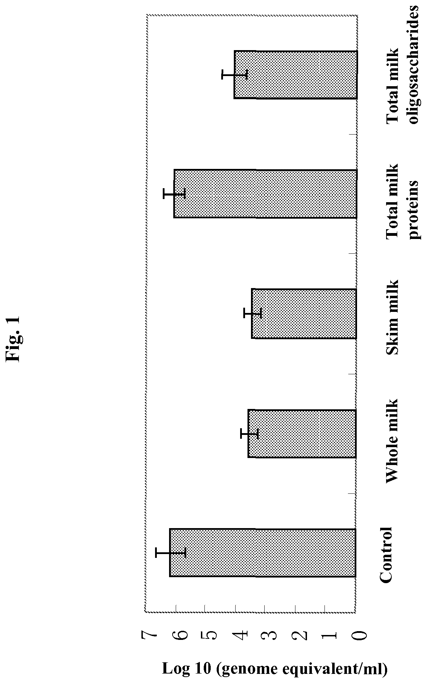

Fig. 1 is a chart showing the inhibitory effects of human milk (5 g/L), skim human milk (5 g/L), total proteins from human milk (5 g/L), and oligosaccharides from human milk (HMO, 5 g/L) on *Clostridium perfringens* growth.

### DETAILED DESCRIPTION OF THE INVENTION

Disclosed herein is a milk-derived oligosaccharide effective in treating infection in an animal. A milk-derived oligosaccharide, i.e., having at least three sugar units, is either a naturally-occurring oligosaccharide found in milk, a fragment of the naturally-occurring oligosaccharide, or a variant thereof that contains a modified (e.g., a sulfated, acetylated, or phosphorylated) sugar unit. This oligosaccharide includes a non-reducing end motif S₁S₂, in which S₁ is fucose, galactose, mannose, or sialic acid (N-acetyl or N-glycolyl) and S₂ is galactose, glucose, mannose, or N-acetylglucosamine. S₁ is linked to S₂ via an α or β glycosidic bond. When S₁ is fucose, the glycosidic bond between S₁ and S₂ preferably is an α1,2, an α1,3, or an α1,4 bond. When it is sialic acid, the glycosidic bond preferably is an α2,3 or an α2,6 bond.

The following tables list exemplary oligosaccharides that naturally occur in human milk:

**Table 1. Fucosyl oligosaccharides**

| | | |
|---|---|---|
| 2'FL | 2-Fucosyllactose | Fucα1,2Galβ1,4Glc |
| LNF-I | Lacto-*N-*fucopentaose I | Fucα1,2Galβ1,3GlcNAcβ1,3Galβ1,4Glc |
| LNF-II | Lacto-*N*-fucopentaose II | Galβ1,3↘ GlcNAcβ1,3Galβ1,4Glc Fucα1,4↗ |
| 3FL | 3-Fucosyllactose | Galβ1,4↘ Glc Fucα1,3↗ |
| LNF-III | Lacto-N-fucopentaose III | Galβ1,4↘ GlcNAcβ1,3Galβ1,4Glc Fucα1,3↗ |
| LDFH-I | Lacto-*N*-difucohexaose I | Fucα1,2Galβ1,3↘ GlcNAcβ1,3Galβ1,4Glc Fucα1,4↗ |
| LDFT | Lactodifucotetraose | Fucα1,2Galβ1,4↘ Glc Fucα1,3↗ |

**Table 2. Nonfucosylated, nonsialylated oligosaccharides**

| | | |
|---|---|---|
| LNT | Lacto-*N*-tetraose | Galβ1,3GlcNAcβ1,3Galβ1,4Glc |
| LNneoT | Lacto-*N*-neotetraose | Galβ1,4GlcNAcβ1,3Galβ1,4Glc |

**Table 3. Sialyl milk oligosaccharide structures**

| | | |
|---|---|---|
| 3'-SL | 3'-Sialyllactose | NANAα2,3Galβ1,4Glc |
| 6'-SL | 6'-Sialyllactose | NANAα2,6Galβ1,4Glc |
| SLNT-c | Sialyllacto-*N*-neotetraose c | NANAα2,6Galβ1,4GlcNAcβ1,3Galβ1,4Glc |
| MSLNH | Monosialyllacto-*N*-hexaose | NANAα2,6Galβ1,4GlcNAc1,6↘ Galβ1,4Glc Galβ1,3GlcNAcβ1,3↗ |
| DSLNH-I | Disialyllacto-*N*-hexaose I | NANAα2,3Galβ1,3GlcNAcβ1,3↘ Galβ1,4Glc NANAα2,6Galβ1,4GlcNAcβ1,6↗ |
| MSLNnH-I | Monosialyllacto-*N*-neohexaose I | NANAα2,6Galβ1,3GlcNAcβ1,3↘ Galβ1,4Glc Galβ1,4GlcNAcβ1,6↗ |
| SLNnH-II | Monosialyllacto-*N*-neohexaose II | Galβ1,4GlcNAcβ1,3↘ Galβ1,4Glc NANAα2,6Galβ1,4GlcNAcβ1,6↗ |
| DSLNnH | Disialyllacto-*N*-neohexaose | NANAα2,6Galβ1,4GlcNAcβ1,3↘ Galβ1,4Glc NANAα2,6Galβ1,4GlcNAcβ1,6↗ |
| DSLNT | Disialyllacto-*N*-tetraose | NANAα2,6↘ GlcNAcβ1,3Galβ1,4Glc NANAα2,3Galβ1,3↗ |
| DSLNH-II | Disialyllacto-*N*-hexaose II | NANAα2,6↘ GlcNAcβ1,3↘ NANAα2,3Galβ1,3↗ Galβ1,4Glc Galβ1,4GlcNAcβ1,6↗ |
| SLNT-a | Sialyllacto-*N*-tetraose a | NANAα2,3Galβ1,3GlcNAcβ1,3 Galβ1,4Glc |
| DSLNH-I | Disialyllacto-*N*-hexaose I | NANAα2,3Galβ1,3GlcNAcβ1,3↘ Galβ1,4Glc NANAα2,6Galβ1,4GlcNAcβ1,6↗ |
| SLNT-b | Sialyllacto-*N*-tetraose b | NANAα2,6↘ GlcNAcβ1,3Galβ1,4Glc Galβ1,3↗ |

**Table 4. Sialyl fucosyl oligosaccharides**

| | | |
|---|---|---|
| 3'-S-3FL | 3'-Sialyl-3-fucosyllactose | NANAα2,3Galβ1,4↘ Glc Fucα1,3↗ |
| DSFLNH | Disialomonofucosyllacto-*N*-neohexaose | NANAα2,6Galβ1,4GlcNAcβ1,6↘ Fucα1,3 ↘ Galβ1,4Glc GlcNAcβ1,3↗ NANAα2,3Galβ1,4↗ |
| MFMSLNO | Monofucosylmonosialyllacto-N-octaose (sialyl Lea) | Galβ1,4GlcNAcβ1,3Galβ1,4GlcNAcβ1,6↘ Galβ1,4Glc NANAα2,3Galβ1,3GlcNAcβ1,3↗ Fucα1,4↗ |
| SLNFH-II | Sialyllacto-N-fucohexaose II | NANAα2,3Galβ1,3GlcNAcβ1,3Galβ1,4Glc Fucα1,4↗ Fucα1,3↗ |
| DSLNFP-II | Disialyllacto-N-fucopentaose II | NANAα2,6↘ NANAα2,3Galβ1,3GlcNAcβ1,3Galβ1,4Glc Fucα1,4↗ |
| MFDLNT | Monofu cosyld isialyllacto-N-tetraose | NANAα2,6↘ NANAα2,3Galβ1,3GlcNAcβ1,3Galβ1,4Glc Fucα1,4↗ |

The milk-derived oligosaccharide described herein can be prepared by conventional methods, e.g., synthesized chemically, purified from milk, or produced in a microorganism. See WO2005/055944. Their anti-infection properties can be confirmed by methods also known in the art. See, e.g., WO2005/055944.

The milk-derived oligosaccharide can be linked to a backbone molecule (e.g., a carbohydrate, a lipid, a nucleic acid, or a peptide) directly or via a linker to form a glycoconjugate. As used herein, "glycoconjugate" refers to a complex containing a sugar moiety associated with a backbone moiety. The sugar and the backbone moieties can be associated via a covalent or noncovalent bond, or via other forms of association, such as entrapment (e.g., of one moiety on or within the other, or of either or both entities on or within a third moiety). The glycoconjugate described herein can contain one type of milk-derived oligosaccharide (i.e., one or more copies of a milk-derived oligosaccharide attached to one backbone molecule). Alternatively, the glycoconjugate contains multiple types of milk-derived oligosaccharides. In one example, the milk-derived oligosaccharide (e.g., lacto-N-fucopentaose I, 2-fucosyllactose, lacto-N-difucohexaose I, lactodifucotetraose, or an acetylated variant thereof) is covalently linked via its reducing end sugar unit to a lipid, a protein, a nucleic acid, or a polysaccharide. Preferably, the reducing end sugar unit is N-acetylglucosamine.

Peptide backbones suitable for making the glycoconjugate described above include those having multiple glycosylation sites (e.g., asparagine, lysine, serine, or threonine residue) and low allergenic potential. Examples include, but are not limited to, amylase, bile salt-stimulated lipase, casein, folate-binding protein, globulin, gluten, haptocorrin, lactalbumin, lactoferrin, lactoperoxidase, lipoprotein lipase, lysozyme, mucin, ovalbumin, and serum albumin.
Typically, a milk-derived oligosaccharide can be covalently attached to a serine or threonine residue via an O-linkage or attached to an asparagine residue via an N-linkage. To form these linkages, the sugar unit at the reducing end of the oligosaccharide is preferably an acetylated sugar unit, e.g., N-acetylgalactosamine, N-acetylglucosamine, and N-acetylmannosamine. An oligosaccharide can be attached to a peptide (e.g., a protein) using standard methods. See, e.g., McBroom et al., Complex Carbohydrates, Part B, 28:212-219, 1972; Yariv et al., Biochem J., 85:383-388, 1962; Rosenfeld et al., Carbohydr. Res., 46:155-158, 1976; and Pazur, Adv. Carbohydr. Chem, Biochem., 39:405-447, 1981.

In one example, a milk-derived oligosaccharide is linked to a backbone molecule via a linker. Exemplary linkers are described in WO2005/055944. The oligosaccharide can be bonded to a linker by an enzymatic reaction, e.g., a glycosyltransferase reaction. A number of glycosyltransferases, including fucosyltransferases, galactosyltransferases, glucosyltransferases, mannosyltransferases, galactosaminyltransferases, sialyltransferases and N-acetylglucosaminyltransferases, can be used to make the glycoconjugate described herein. More details about these glycosyltransferases can be found in U.S. Patent Nos: 6,291,219; 6,270,987; 6,238,894; 6,204,431; 6,143,868; 6,087,143; 6,054,309; 6,027,928; 6,025,174; 6,025,173; 5,955,282; 5,945,322; 5,922,540; 5,892,070; 5,876,714; 5,874,261; 5,871,983; 5,861,293; 5,859,334; 5,858,752; 5,856,159; and 5,545,553.

One or more of the above described milk-derived oligosaccharides and/or glycoconjugates can be mixed with an animal foodstuff to form a food composition (which is not naturally occurring) for animal consumption. The term "an animal foodstuff" used herein refers to a food product or food supplement for animal consumption, not for human consumption. In one example, the animal foodstuff is a commercially available animal food, e.g., those provided by Purina Mills. The animal food composition, which may be free of a milk product or milk by-product, can be in any suitable form, such as a biscuit, a cracker, a gel, a granule, a kibble, a liquid, a nugget, a paste, a pellet, a powder, or a syrup.

One or more of the milk-derived oligosaccharides and/or glycoconjugates described herein can also be mixed with a pharmaceutically acceptable carrier to form a veterinary pharmaceutical composition. Optionally, the pharmaceutical composition further contains one or more additional therapeutic agents, e.g., an analgesic, an antibiotic, an anti-inflammatory agent, or a probiotic. An "acceptable carrier" is compatible with the active ingredient(s) of the composition, i.e., the milk-derived oligosaccharide(s) or the glycoconjugate(s), preferably capable of stabilizing the active ingredient(s), and not deleterious to the animal to be treated. Suitable carriers include microcrystalline cellulose, mannitol, glucose, polyvinylpyrrolidone, and starch, or a combination thereof. Methods for preparing a veterinary pharmaceutical composition are known in the art. See e.g., US Patent No. 5,958,464.

A veterinary pharmaceutical composition can be administered buccally, nasally, orally, parenterally, rectally, topically, vaginally, or via an implanted reservoir, inhalation spray, or direct infusion into the GI tract or stomach.

A veterinary pharmaceutical composition for oral administration can be any orally acceptable dosage form including, but not limited to bolus, capsule, dispersion, electuary, emulsion and aqueous suspension, gel, granule, paste, pellet, powder, slurry, solution, syrup, and tablet. An orally administered veterinary composition can include binders, lubricants, inert diluents, lubricating, surface active or dispersing agents, flavoring agents, and humectants. In the case of a tablets/capsules, carriers which are commonly used include lactose and corn starch. Lubricating agents, such as magnesium stearate, are also typically added. Tablets/capsules may optionally be coated or formulated so as to provide sustained, delayed, or controlled release of the active ingredient therein. When aqueous suspensions or emulsions are administered orally, the active ingredient can be suspended or dissolved in an oily phase combined with emulsifying or suspending agents. A nasal aerosol or inhalation composition can be prepared according to techniques well known in the art of pharmaceutical formulation.

The particular formulation and dosage depends on the animal species; the administration route; the characteristics of the formulation; age/size of the animal; and the nature of the animal's infection, if any. In some cases, the dosage will be at a concentration similar to that found for similar oligosaccharides present in human breast milk.

Also disclosed herein is a method of using the milk-derived oligosaccharide and the glycoconjugate for treating animal infection, i.e., a detrimental colonization of a microbe in a host animal, resulting in an illness in the animal. The term "treating" used herein refers to the application or administration of a composition including one or more active agents to an animal, who has infection, a symptom of the infection, or a predisposition toward the infection, with the purpose to cure, heal, alleviate, relieve, alter, remedy, ameliorate, improve, or affect the infection, the symptoms of the infection, or the predisposition toward the infection. To treat infection, an effective amount of the milk-derived oligosaccharide or the glycoconjugate can be administered to an animal that is infected by a pathogenic microbe or at risk for infection via conventional routes. An "effective amount" is the amount of each active agent required to confer therapeutic effect on the animal, either alone or in combination with one or more other active agents. Effective amounts vary, as recognized by those skilled in the art, depending on route of administration, excipient usage, and co-usage with other active agents.

The food or veterinary pharmaceutical composition described herein is useful for treating an infection caused by *Clostridium perfringens*
Animals that can be treated by the method include cat, dog, marsupial, primate, rabbit, rodent (e.g., chinchilla, gerbil, guinea pig, mouse, and rat), ungulate (for example, bovid (e.g., African Buffalo, bison, cow, goat, ox, sheep, water buffalo, or yak), camelid (e.g., alpaca, camel, and llama), deer, equine (e.g., donkey, horse, and mule), and pig), weasel (e.g., ermine, ferret, mink, and weasel), chicken, dove, duck, finch, goose, guinea fowl, hummingbird, parrot (e.g., cockatiel, cockatoo, lorikeet, lovebird, macaw, parakeet), pheasant (e.g., partridge, pheasant, peafowl, and quail), pigeon, raptor, ratite (e.g., emu, kiwi, ostrich, and rhea), swan, turkey, anchovy, barramundi, bass, catfish, cod, cyprinid (e.g., carp, goldfish, koi, and minnow), eel, flounder, herring, mackerel, mullet, perch, pollack, salmon, sardine, sturgeon, tilapia, trout, tuna, frog, toad, and reptile (e.g., crocodilian, lizard, snake, and turtle).

An animal in need of the treatment can be fed on the oligosaccharide/glycoconjugate together with water or its daily food (e.g., fruit, raw meat, hay, or a commercial animal food). For example, when a livestock animal (e.g., cow, goat, horse, pig, and sheep) is to be treated, the oligosaccharide can be mixed with fodder (e.g., alfalfa, barley, corn, grain, grass, hay, legume, millet, nut, oat, rice, rye, seaweed, seed, sorghum, soybean, straw, and wheat) and provided during regular feedings. Alternatively, the animal is fed with the animal food compositions described above or administered with the pharmaceutical composition also described above via conventional routes.

To reduce the risk of contracting an infection in an animal, the milk-derived oligosaccharide or glycoconjugate is preferably co-used with a probiotic (i.e., a dietary supplements of a live bacterium or yeast that benefits the animal to be treated) or a prebiotic (i.e., a non-digestible food ingredient that beneficially affects an animal to be treated by selectively stimulating the growth and/or activity of beneficial bacteria in the digestive tract). Prebiotics are typically oligosaccharides, including fructooligosaccharides, xylooligosaccharides, polydextrose, galactooligosaccharides, and mannooligosaccharides.

Without further elaboration, it is believed that one skilled in the art can, based on the above description, utilize the present invention to its fullest extent. The example provided below, showing the inhibitory effect of oligosaccharides of human milk against C. *perfringens* proliferation, is therefore to be construed as merely illustrative, and not limitative of the remainder of the disclosure in any way whatsoever. All publications cited herein are incorporated by reference.

Anaerobic batch cultures were used to study the effect of human milk oligosaccharides on growth of C. *perfringens,* a pathogen capable of causing severe infections in farm animals (e.g., cow, pig, horse, poultry such as chicken, ostrich, and emu, sheep, rabbit, goat, hog, cattle, mink), birds, dogs, and cats, *etc..*

Briefly, C. *perfringens* cells were cultured in a medium (10 ml) in the presence of (a) whole human milk, (b) skim human milk, (c) total proteins isolated from human milk, or (d) oligosaccharides purified from human milk. The oligosaccharides and other milk fractions were prepared following the method described in Newburg et al., Journal of Infectious Diseases 1990; 162: 1075-1080, and Yolken et al., Journal of Clinical Investigation 1992; 90: 1984-1991.
*C. perfringens* cells, cultured in the same medium without any carbohydrate addition, were used as a blank control.

Twenty-four hours later, the cells in each group were collected and subjected to a routine procedure (see, e.g., Henriksen, et al., Avian Dis 2009; 53: 441-8 and Wise et al., Appl Environ Microbiol 2005; 71: 3911-6) to determine the copy number of the 16S rRNA gene, which is used as a measure of the number of *C. perfringens* cells. As shown in Fig. 1., the copy number of the 16S rRNA gene in the *C. perfringens* cells incubated with whole human milk, skim human milk, or human milk oligosaccharides were much lower than that in the C. *perfringens* cells incubated with human milk proteins or in the control cells. This indicates that the C. *perfringens* cells treated with whole human milk, skim human milk, or human milk oligosaccharides multiplied much less than those untreated or treated with human milk proteins. In sum, the result obtained from this study demonstrates that human milk fractions containing oligosaccharides and the isolated oligosaccharides per se are effective in suppressing C. *perfringens* growth. Thus, human milk oligosaccharides are effective in treating infections caused by *C. perfringens.*

All of the features disclosed in this specification may be combined in any combination.

## Claims

1. A food composition for use in treating an infection in an animal, comprising
an animal foodstuff, and
a first milk-derived oligosaccharide or a glycoconjugate containing the first oligosaccharide, the first oligosaccharide including a first sugar unit linked to a second sugar unit, wherein the first sugar unit, located at a first non-reducing end, is fucose, galactose, mannose, or sialic acid and the second sugar unit is galactose, glucose, mannose, or N-acetylglucosamine,
wherein the infection is caused by *Clostridium perfringens.*

2. A veterinary pharmaceutical composition for use in treating an infection in an animal, comprising
a first milk-derived oligosaccharide or a glycoconjugate containing the first oligosaccharide, the first oligosaccharide including a first sugar unit linked to a second sugar unit, wherein the first sugar unit, located at a non-reducing end, is fucose, galactose, mannose, or sialic acid and the second sugar unit is galactose, glucose, mannose, or N-acetylglucosamine and
a pharmaceutically acceptable carrier;
wherein the pharmaceutical composition is formulated for animal use and wherein the infection is caused by *Clostridium perfringens.*

3. The composition for use according to claim 1 or 2, wherein the first oligosaccharide is an oligosaccharide found in human milk.

4. The composition for use according to claim 3, wherein the first sugar unit is fucose or sialic acid.

5. The composition for use according to claim 4, wherein the first oligosaccharide is 2'-fucosyllactose, lacto-*N*-fucopentaose I, 3'-sialyllactose, 6'-sialyllactose, sialyllacto-*N-*neotetraose c, sialyllacto-*N*-tetraose a, or a variant thereof that is identical to one of the oligosaccharides except that the glucose at the reducing end is replaced with N-acetylglucosamine.

6. The composition for use according to claim 3 or claim 4, wherein the first oligosaccharide includes a second non-reducing end.

7. The composition for use according to claim 6, wherein the sugar unit at the second non-reducing end is selected from the group consisting of fucose, galactose, and sialic acid.

8. The composition for use according to claim 7, wherein the first oligosaccharide is lacto-*N-*difucohexaose I, lactodifucotetraose, lacto-*N-*fucopentaose II, 3-fucosyllactose, lacto-*N*-fucopentaose III, 3'-sialyl-3-fucosyllactose, disialomonofucosyllacto-*N*-neohexaose, monofucosylmonosialyllacto-*N*-octaose, sialyllacto-*N-*fucohexaose II, disialyllacto-*N-*fucopentose II, monofucosyldisialyllacto-*N-*tetraose, monosialyllacto-*N*-hexaose, monosialyllacto-*N-*neohexaose I, monosialyllacto-*N*-neohexaose II, sialyllacto-*N-*tetraose b, disialyllacto-*N-*hexaose I, disialyllacto-*N-*neohexaose, disialyllacto-*N-*tetraose, disialyllacto-*N-*hexaose II, or a variant thereof that is identical to one of the oligosaccharides except that the glucose at the reducing end is replaced with N-acetylglucosamine.

9. The composition for use according to claim 1 or 2, further comprising a second milk-derived oligosaccharide including a first sugar unit linked to a second sugar unit, the first sugar unit, located at a first non-reducing end, being fucose, galactose, mannose, or sialic acid and the second sugar unit being galactose, glucose, mannose, or N-acetylglucosamine, wherein the second oligosaccharide is different from the first oligosaccharide.

10. The composition for use according to claim 9, wherein the second oligosaccharide is 2'-fucosyllactose, lacto-*N-*fucopentaose I, 3'-sialyllactose, 6'-sialyllactose, sialyllacto-*N-*neotetraose c, sialyllacto-*N-*tetraose a, lacto-*N-*difucohexaose I, lactodifucotetraose, lacto-*N*-fucopentaose II, 3-fucosyllactose, lacto-*N*-fucopentaose III, 3'-sialyl-3-fucosyllactose, disialomonofucosyllacto-*N-*neohexaose, monofucosylmonosialyllacto-*N*-octaose, sialyllacto-*N*-fucohexaose II, disialyllacto-*N-*fucopentose II, monofucosyldisialyllacto-*N*-tetraose, monosialyllacto-*N-*hexaose, monosialyllacto-*N-*neohexaose I, monosialyllacto-*N-*neohexaose II, sialyllacto-*N*-tetraose b, disialyllacto-*N-*hexaose I, disialyllacto-*N-*neohexaose, disialyllacto-*N-*tetraose, disialyllacto-*N-*hexaose II, or a variant thereof that is identical to one of the oligosaccharides except that the glucose at the reducing end is replaced with N-acetylglucosamine.

11. The composition for use according to any of claims 3-10, comprising the glycoconjugate to which the first oligosaccharide and the second oligosaccharide, if any, are attached.

12. The composition for use according to claim 11, wherein in the glycoconjugate, the oligosaccharide(s) is conjugated with a carbohydrate, a lipid, or a peptide.

13. The composition for use according to any of claims 1-12, wherein the composition is free of a milk product or milk by-product.

14. The composition for use according to any of claims 1-13, wherein the animal is preferably a mammal or a bird, still preferably a mammal selected from the group consisting of cat, cattle, cow, dog, goat, horse, pig, rabbit, rodent, mink, and sheep or a bird selected from the group consisting of chicken, duck, goose, turkey, ostrich, emu, swan, peafoul, pheasant, partridge, and guineafowl.

## Patentansprüche

1. Eine Nahrungsmittelzusammensetzung zur Verwendung in der Behandlung einer Infektion in einem Tier umfassend ein Tierfuttermittel und
ein erstes aus Milch gewonnenes Oligosaccharid oder ein Glycokonjugat enthaltend das erste Oligosaccharid, das erste Oligosaccharid beinhaltend eine erste Zuckereinheit verbunden mit einer zweiten Zuckereinheit, wobei die erste Zuckereinheit, lokalisiert an einem ersten nicht-reduzierenden Ende, Fucose, Galaktose, Mannose oder Sialsäure ist und die zweite Zuckereinheit Galaktose, Glukose, Mannose oder N-Acetylglucosamin ist,
wobei die Infektion durch *Clostridium perfringens* verursacht wird.

2. Eine veterinärmedizinische, pharmazeutische Zusammensetzung zur Verwendung in der Behandlung einer Infektion in einem Tier umfassend
ein erstes aus Milch gewonnenes Oligosaccharid oder ein Glycokonjugat enthaltend das erste Oligosaccharid, das erste Oligosaccharid beinhaltend eine erste Zuckereinheit verbunden mit einer zweiten Zuckereinheit, wobei die erste Zuckereinheit, lokalisiert an einem ersten nicht-reduzierenden Ende, Fucose, Galaktose, Mannose oder Sialsäure ist und die zweite Zuckereinheit Galaktose, Glukose, Mannose oder N-Acetylglucosamin ist und ein pharmazeutisch verträglicher Träger,
wobei die pharmazeutische Zusammensetzung für den tierischen Gebrauch formuliert ist und wobei die Infektion durch *Clostridium perfringens* verursacht wird.

3. Die Zusammensetzung zur Verwendung nach Anspruch 1 oder 2, wobei das erste Oligosaccharid ein in humaner Milch gefundenes Oligosaccharid ist.

4. Die Zusammensetzung zur Verwendung nach Anspruch 3,, wobei die erste Zuckereinheit Fucose oder Sialsäure ist.

5. Die Zusammensetzung zur Verwendung nach Anspruch 4, wobei das erste Oligosaccharid 2'-Fucosyllactose, Lacto-*N-*fucopentaose I, 3'-Sialyllactose, 6'-Sialyllactose, Sialyllacto-*N-*neotetraose c, Sialyllacto-*N-*tetradose a oder eine Variante davon ist, die identisch ist zu einem der Oligosaccharide, außer dass die Glukose am reduzierenden Ende durch N-Acetylglucosamin ersetzt ist.

6. Die Zusammensetzung zur Verwendung nach Anspruch 3 oder 4, wobei das erste Oligosaccharid ein zweites nicht-reduzierendes Ende beinhaltet.

7. Die Zusammensetzung zur Verwendung nach Anspruch 6, wobei die Zuckereinheit am zweiten nicht-reduzierenden Ende ausgewählt ist aus der Gruppe bestehend aus Fucose, Galaktose und Sialsäure.

8. Die Zusammensetzung zur Verwendung nach Anspruch 7, wobei das erste Oligosaccharid Lacto-*N-*difucohexaose I, Lactodifucotetraose, Lacto-*N-*fucopentaose II, 3-Fucosyllactose, Lacto-*N-*fucopentaose III, 3'-Sialyl-3-fucosyllactose, Disialomonofucosyllacto-*N-*neohexaose, Monofucosylmonosialyllacto-*N-*octaose, Sialyllacto-*N-*fucohexaose II, Disialyllacto-*N-*fucopentose II, Monofucosyldisialyllacto-*N-*tetraose, Monosialyllacto-*N-*hexaose, Monosialyllacto-*N-*neohexaose I, Monosialyllacto-*N-*neohexaose II, Sialyllacto-*N-*tetraose b, Disialyllacto-*N-*hexaose I, Disialyllacto-*N-*neohexose, Disialyllacto-*N-*tetraose, Disialyllacto-*N-*hexaose II oder eine Variante davon, die identisch ist mit einem der Oligosaccharide, außer dass die Glukose an dem reduzierenden Ende durch N-Acetylglucosamin ersetzt ist.

9. Die Zusammensetzung zur Verwendung nach Anspruch 1 oder 2, ferner umfassend ein zweites aus der Milch gewonnenes Oligosaccharid beinhaltend eine erste Zuckereinheit verbunden mit einer zweiten Zuckereinheit, die erste Zuckereinheit lokalisiert an einem ersten nicht-reduzierenden Ende ist Fucose, Galaktose, Mannose oder Sialsäure und die zweite Zuckereinheit ist Galaktose, Glukose, Mannose oder N-Acetylglucaosamin, wobei sich das zweite Oligosaccharid vom ersten Oligosaccharid unterscheidet.

10. Die Zusammensetzung zur Verwendung nach Anspruch 9, wobei das zweite Oligosaccharid 2'-Fucosyllactose, Lacto-*N-*fucopentaose I, 3'-Sialyllactose, 6'-Sialyllactose, Sialyllacto-*N-*neotetraose c, Sialyllacto-*N-*tetraose a, Lacto-*N-*difucohexaose I, Lactodifucotetraose, Lacto-N-fucopentaose II, 3-Fucosyllactose, Lacto-*N-*fucopentaose III, 3'-Sialyl-3-fucosyllactose, Disialomonofucosyllacto-*N-*neohexaose, Monofucosylmonosialyllacto-*N-*ocotaose, Sialyllacto-*N-*fucohexaose II, Disialyllacto-*N-*fucopentose II, Monofucosyldisialyllacto-*N-*tetraose, Monosialyllacto-*N-*hexaose, Monosialyllacto-*N-*neohexaose I, Monosialyllacto-*N-*neohexaose II, Sialyllacto-*N-*tetraose b, Disialyllacto-*N-*hexaose I, Disialyllacto-*N-*neohexaose, Disialyllacto-*N-*tetraose, Disialyllacto-*N-*hexaose II oder eine Variante davon ist, die identisch ist mit einem der Oligosaccharide außer dass die Glukose am reduzierenden Ende durch N-Acetylglucosamin ersetzt ist.

11. Die Zusammensetzung zur Verwendung nach einem der Ansprüche 3 bis 10 umfassend das Glycokonjugat an das das erste Oligosaccharid und das zweite Oligosaccharid, falls ein solches vorhanden ist, gebunden sind.

12. Die Zusammensetzung zur Verwendung nach Anspruch 11, wobei in dem Glycokonjugat das/die Oligosaccharid(e) mit einem Kohlenhydrat, einem Lipid oder einem Peptid konjugiert ist.

13. Die Zusammensetzung zur Verwendung nach einem der Ansprüche 1 bis 12, wobei die Zusammensetzung frei von einem Milchprodukt oder Milch-Nebenprodukt ist.

14. Die Zusammensetzung zur Verwendung nach einem der Ansprüche 1 bis 13, wobei das Tier bevorzugt ein Säugetier oder ein Vogel, mehr bevorzugt ein Säugetier ausgewählt aus der Gruppe bestehend aus Katze, Rind, Kuh, Hund, Ziege, Pferd, Schwein, Hase, Nagetier, Nerz und Schaf oder ein Vogel ausgewählt aus der Gruppe bestehend aus Huhn, Ente, Gans, Truthahn, Strauß, Emu, Schwan, Pfau, Fasan, Rebhuhn und Perlhuhn ist.

## Revendications

1. Composition alimentaire destinée à être utilisée dans le traitement d'une infection chez un animal, comprenant des aliments pour animaux, et
un premier oligosaccharide dérivé du lait ou un glycoconjugué contenant le premier oligosaccharide, le premier oligosaccharide comprenant une première unité de sucre liée à une seconde unité de sucre, dans laquelle la première unité de sucre, localisée au niveau d'une première extrémité non réductrice, est du fucose, du galactose, du mannose, ou de l'acide sialique et la seconde unité de sucre est du galactose, du glucose, du mannose, ou de la N-acétylglucosamine,
dans laquelle l'infection est provoquée par *Clostridium perfringens.*

2. Composition pharmaceutique vétérinaire destinée à être utilisée dans le traitement d'une infection chez un animal, comprenant
un premier oligosaccharide dérivé du lait ou un glycoconjugué contenant le premier oligosaccharide, le premier oligosaccharide comprenant une première unité de sucre liée à une seconde unité de sucre, dans laquelle la première unité de sucre, localisée au niveau d'une première extrémité non réductrice, est du fucose, du galactose, du mannose, ou de l'acide sialique et la seconde unité de sucre est du galactose, du glucose, du mannose, ou de la N-acétylglucosamine et
un support pharmaceutiquement acceptable ;
dans laquelle la composition pharmaceutique est formulée pour un usage animal et dans laquelle l'infection est provoquée par *Clostridium perfringens.*

3. Composition destinée à être utilisée selon la revendication 1 ou 2, dans laquelle le premier oligosaccharide est un oligosaccharide trouvé dans le lait humain.

4. Composition destinée à être utilisée selon la revendication 3, dans laquelle la première unité de sucre est du fucose ou de l'acide sialique.

5. Composition destinée à être utilisée selon la revendication 4, dans laquelle le premier oligosaccharide est le 2'-fucosyl-lactose, le lacto-*N*-fucopentaose I, le 3'-sialyl-lactose, le 6'-sialyl-lactose, le sialyl-lacto-*N*-néotétraose c, le sialyl-lacto-*N*-tétraose a, ou l'un de leurs variants qui est identique à l'un des oligosaccharides à l'exception que le glucose à l'extrémité réductrice est remplacé par de la N-acétylglucosamine.

6. Composition destinée à être utilisée selon la revendication 3 ou la revendication 4, dans laquelle le premier oligosaccharide comprend une seconde extrémité non réductrice.

7. Composition destinée à être utilisée selon la revendication 6, dans laquelle l'unité de sucre au niveau de la seconde extrémité non réductrice est choisie dans le groupe constitué du fucose, du galactose, et de l'acide sialique.

8. Composition destinée à être utilisée selon la revendication 7, dans laquelle le premier oligosaccharide est le lacto-*N*-difucohexaose I, le lactodifucotétraose, le lacto-N-fucopentaose II, le 3-fucosyl-lactose, le lacto-*N*-fucopentaose III, le 3'-sialyl-3-fucosyl-lactose, le disialo-monofucosyl-lacto-*N*-néohexaose, le monofucosyl-monosialyl-lacto-*N*-octaose, le sialyl-lacto-*N*-fucohexaose II, le disialyl-lacto-*N-*fucopentose II, le monofucosyl-disialyl-lacto-*N*-tétraose, le monosialyl-lacto-*N-*hexaose, le monosialyl-lacto-*N*-néohexaose I, le monosialyl-lacto-*N*-néohexaose II, le sialyl-lacto-*N*-tétraose b, le disialyl-lacto-*N*-hexaose I, le disialyl-lacto-*N-*néohexaose, le disialyl-lacto-*N*-tétraose, le disialyl-lacto-*N*-hexaose II, ou l'un de leurs variants qui est identique à l'un des oligosaccharides à l'exception que le glucose à l'extrémité réductrice est remplacé par de la N-acétylglucosamine.

9. Composition destinée à être utilisée selon la revendication 1 ou 2, comprenant en outre un second oligosaccharide dérivé du lait comprenant une première unité de sucre liée à une seconde unité de sucre, la première unité de sucre, localisée au niveau d'une première extrémité non réductrice, étant du fucose, du galactose, du mannose, ou de l'acide sialique et la seconde unité de sucre étant du galactose, du glucose, du mannose, ou de la N-acétylglucosamine, dans laquelle le second oligosaccharide est différent du premier oligosaccharide.

10. Composition destinée à être utilisée selon la revendication 9, dans laquelle le second oligosaccharide est le 2'-fucosyl-lactose, le lacto-*N*-fucopentaose I, le 3'-sialyl-lactose, le 6'-sialyl-lactose, le sialyl-lacto-*N*-néotétraose c, le sialyl-lacto-*N*-tétraose a, le lacto-*N*-difucohexaose I, le lactodifucotétraose, le lacto-*N*-fucopentaose II, le 3-fucosyl-lactose, le lacto-*N*-fucopentaose III, le 3'-sialyl-3-fucosyl-lactose, le disialo-monofucosyl-lacto-*N*-néohexaose, le monofucosyl-monosialyl-lacto-*N*-octaose, le sialyl-lacto-*N*-fucohexaose II, le disialyl-lacto-*N*-fucopentose II, le monofucosyl-disialyl-lacto-*N*-tétraose, le monosialyl-lacto-*N*-hexaose, le monosialyl-lacto-*N-*néohexaose I, le monosialyl-lacto-*N*-néohexaose II, le sialyl-lacto-*N*-tétraose b, le disialyl-lacto-*N*-hexaose I, le disialyl-lacto-*N*-néohexaose, le disialyl-lacto-*N-*tétraose, le disialyl-lacto-*N*-hexaose II, ou l'un de leurs variants qui est identique à l'un des oligosaccharides à l'exception que le glucose à l'extrémité réductrice est remplacé par de la N-acétylglucosamine.

11. Composition destinée à être utilisée selon l'une quelconque des revendications 3 à 10, comprenant le glycoconjugué auquel le premier oligosaccharide et le second oligosaccharide, éventuellement, sont fixés.

12. Composition destinée à être utilisée selon la revendication 11, dans laquelle dans le glycoconjugué, le ou les oligosaccharides sont conjugués avec un glucide, un lipide, ou un peptide.

13. Composition destinée à être utilisée selon l'une quelconque des revendications 1 à 12, où la composition est dépourvue de produit laitier ou de sous-produit laitier.

14. Composition destinée à être utilisée selon l'une quelconque des revendications 1 à 13, dans laquelle l'animal est de préférence un mammifère ou un oiseau, de façon encore préférée un mammifère choisi dans le groupe constitué du chat, bovin, vache, chien, chèvre, cheval, porc, lapin, rongeur, vison, et mouton ou un oiseau choisi dans le groupe constitué du poulet, canard, oie, dinde, autruche, émeu, cygne, paon, faisan, perdrix, et pintade.
